# FASCICULE DE BREVET EUROPEEN

(11) **EP 0 817 598 B1**
(45) Date de publication et mention de la délivrance du brevet: **14.07.1999**
(21) Numéro de dépôt: 96901944.7
(22) Date de dépôt: 28.02.1996
(51) Int. Cl.: A61F 2/04, A61L 27/00

(54) **PROTHESE POUR S'OPPOSER AU REFLUX GASTRIQUE DANS L' OESOPHAGE**
PROTHESE, DIE SICH DEM GASTRISCHEN RÜCKFLUSS IN DIE SPEISERÖHRE WIDERSETZT
PROSTHESIS FOR PREVENTING GASTRO-OESOPHAGEAL REFLUX

(30) Priorité: 28.03.1995 CH 86695
(43) Date de publication de la demande: 14.01.1998
(73) Titulaire: Biomedix SA, 1201 Genève (CH)
(72) Inventeur: Godin, Norman, Dr., 1201 Genève (CH)
(86) Numéro de dépôt international: IB9600149
(87) Numéro de publication internationale: WO9629954

(56) Documents cités:
- EP-A- 0 275 535
- WO-A-89/05127
- WO-A-91/01117
- FR-A- 1 576 374
- FR-A- 2 513 111
- US-A- 4 846 836
- DATABASE WPI Week 9131 23 Octobre 1991 Derwent Publications Ltd., London, GB; AN 91-265598 XP002008482 & SU,A,1 600 785 (ALMA MED INST) , 23 Octobre 1990

## Description

La présente invention se rapporte à une prothèse pour s'opposer au reflux gastrique dans l'oesophage, comportant un conduit tubulaire en un matériau polymère bio-compatible résistant à l'acide gastrique, dont une première extrémité est fixée en amont de l'estomac et dont l'autre extrémité, libre, se situe en aval de la première extrémité du conduit.

Une telle prothèse, décrite dans le WO 91 01117, présente la forme d'une valve dont la section de passage d'une ouverture est contrôlée par des moyens élastiques. La section du conduit est déformée progressivement de façon permanente pour resserrer la paroi de ce conduit à l'une de ses extrémités de sorte que, en postion d'ecartement maximum de la paroi à cette extrémité, une ouverture se forme qui correspond sensiblement à celle de l'autre extrémité fixée à la paroi de l'oesophage. Il s'agit donc d'une valve dont l'ouverture est provoquée par une force susceptible de vaincre la force élastique qui tend à la maintenir fermée. Un tel concept implique un organe relativement rigide pour permettre sa fermeture en l'absence de force susceptible de l'ouvrir, cette force étant générée par la pression péristaltique exercée sur le bol alimentaire par l'oesophage.

L'inconvénient d'une telle solution est d'aller à l'encontre de ce qui se passe naturellement, c'est-à-dire de faire en sorte que la pression péristaltique doive ouvrir la valve. Dans certaines conditions, par exemple en avalant des aliments sans qu'ils aient été suffisamment mastiqués, la force nécessaire à la déglutition se trouve accrue et si, de plus, il faut que l'oesophage fournisse encore une force supplémentaire pour ouvrir la valve, on risque soit d'arriver à un coincement du bol alimentaire, soit de provoquer des douleurs soit encore les deux.

Le but de la présente invention est de remédier, au moins en partie, aux inconvénients de la solution susmentionnée.

On a constaté qu'un autre concept, sensiblement différent du précédent, bien qu'à première vue il puisse paraître lui ressembler, permet d'atteindre un résultat équivalent sans présenter les inconvénients susmentionnés. Ce concept est basé sur un simple élément tubulaire souple de section sensiblement constante destiné à prolonger l'oesophage jusque dans l'estomac. On sait que l'estomac a une forme dissymétrique par rapport à l'axe de l'oesophage de sorte que, en cas de reflux gastrique, la pression exercée a une direction oblique par rapport à l'axe de l'oesophage. Par conséquent, si on prolonge l'oesophage par un tube souple s'étendant sur une certaine longueur dans l'estomac, en cas de reflux gastrique le tube souple est écrasé par la pression oblique et empêche la sortie d'acide gastrique dans l'oesophage.

La présente invention a, par conséquent, pour objet une prothèse pour s'opposer au reflux gastrique dans l'oesophage selon la revendication 1.

Outre les avantages susmentionnés, la prothèse objet de l'invention est plus facile à introduire dans l'oesophage à l'aide d'un endoscope en raison de sa plus grande souplesse; elle est aussi plus facile à fixer pour les mêmes raisons. Etant donné que la prothèse tubulaire est normalement ouverte, contrairement à la valve susmentionnée qui est normalement fermée, la force exercée sur la fixation de cette valve à la paroi de l'oesophage ou à une hernie hiatale est très fortment réduite puisque seule la force de glissement, le long de la paroi de cette prothèse tubulaire, intervient.

Le dessin annexé illustre, schématiquement et à titre d'exemple, une forme d'exécution et une variante de la prothèse objet de la présente invention.
La figure 1 est une vue en coupe d'un estomac et de l'oesophage avec la prothèse objet de l'invention
La figure 2 est une vue en coupe de la prothèse de la figure 1.
La figure 3 est une vue en coupe d'une variante de la prothèse de la figure 2.

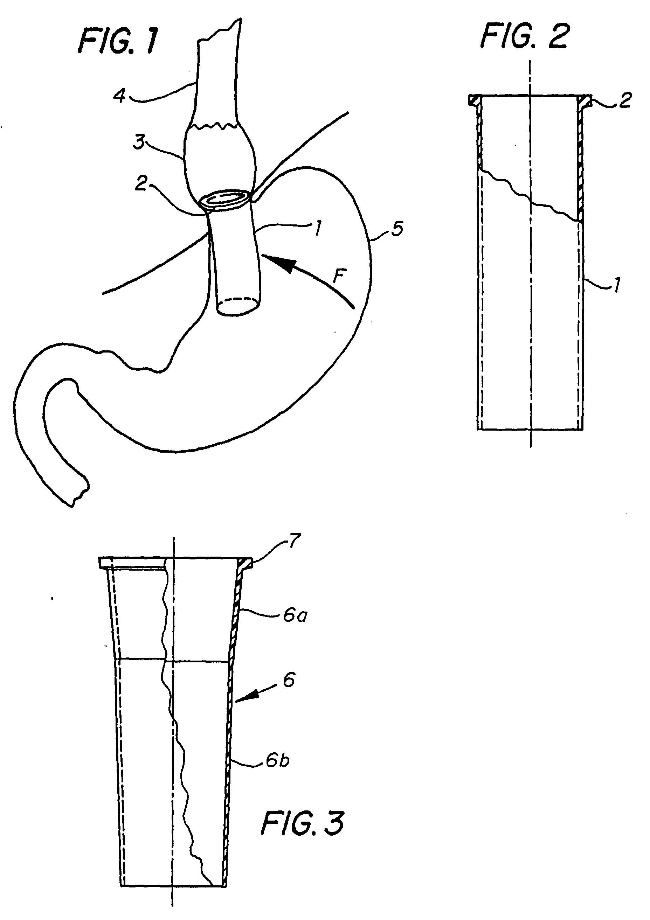

La prothèse illustrée par les figure 1 et 2 est constituée par un simple tube souple 1 en un polymère biocompatible susceptible de résister à l'acidité de l'estomac. On peut envisager divers polymères tels que le silicone spécifiquement destiné aux applications médicales, le polyuréthane aussi destiné à de telles applications, le PSE, ou encore certains élastomères sans que cette liste soit limitative.

Les dimensions de ce tube sont par exemple, d'environ 25 à 30 mm de diamètre et peuvent varier suivant le diamètre de la hernie hiatale ou de l'oesophage où la prothèse est implantée et de 5 à 10 cm de longueur environ, la majeure partie de cette longueur étant destinée à se trouver dans l'estomac. Quant à l'épaisseur de sa paroi, elle est sensiblement dépendante de la nature du polymère qui détermine sa souplesse, mais elle varie généralement de 0,2 à 0,6 mm environ. Il faut, en effet que ce tube soit souple, mais qu'il offre tout de même une résistance suffisante à la pression pour qu'il ne se retourne pas lorsque la pression ne dépasse pas la pression de reflux. Par contre, à des pressions sensiblement plus élevées, correspondant à celles exercées lors des vomissements, il est préférable que le tube puisse se retourner. Dans ce cas, qui demeure très exceptionnel, l'élasticité du tube peut lui permettre de reprendre sa postion normale grâce au péristaltisme ou une intervention endoscopique peut être nécessaire pour remettre le tube dans sa position correcte.

Comme illustré par la figure 1, en cas de surpression dans l'estomac 5, la force s'exerce non pas dans l'axe de la prothèse tubulaire 1 mais à peu près comme indiqué par la flèche F, c'est-à-dire qu'elle a une direction oblique par rapport au tube 1 de sorte que si la paroi de celui-ci est suffisamment souple, il s'écrasera contre la paroi de l'estomac 5 et empêchera l'acide gastrique de sortir et d'atteindre la muqueuse de l'oesophage.

Sur la figure 1, la prothèse tubulaire présente une collerette 2 fixée par des points de suture ou des agrafes à la base d'une hernie hiatale 3 constituant le facteur essentiel facilitant le reflux gastrique: elle pourrait aussie être fixée à la base de l'oesophage 4. Les points de suture ou la pose d'agrafes en métal ou nylon peuvent être réalisées par voie endoscopique à l'aide de matériel destiné à cet effet.

La fabrication de la prothèse tubulaire 1 peut être réalisée par différentes techniques suivant les matériaux utilisés, en particulier leur fluidité et l'épaisseur de la paroi du tube. On peut la réaliser par injection, par extrusion ou par une technique de trempage répété jusqu'à l'obtention de l'épaisseur désirée et connue sous la dénomination de "solvent casting". Dans le cas d'injection, il est souhaitable de prévoir une légère conicité de l'ordre de 1° ou 2° pour le démoulage.

La variante illustrée par la figure 3 montre une prothèse tubulaire injectée 6, comportant une collerette 7 plus épaisse à une extrémité afin de solidifier la paroi à l'endroit de la fixation.

Dans le cas des figures 1 et 2, la prothèse tubulaire obtenue par moulage par injection a une très légère conicité de 1° pour faciliter le démoulage.

Dans le cas de la variante de la figure 3, la prothèse tubulaire 6 comporte deux segments, un segment supérieur, 6a, adjacent à la collerette de fixation 7 et qui, dans cet exemple, a 25 mm de longeur, 3° de conicité et une épaiseur de 0,5 mm, et un segment inférieur 6b qui, dans cet exemple, a 55 mm de longueur, 1° de conicité et une épaisseur de 0,3 mm. C'est ce segment inférieur 6b qui est destiné à s'etendre dans l'estomac 5 (figure 1) et à s'écraser en cas de surpression due au reflux gastrique.

La prothèse objet de l'invention permet de remplir les mêmes fonctions que la valve décrite dans le WO 91 01117 sans en présenter les inconvénients et sa très grande souplesse en facilite l'introduction et la fixation par voie endoscopique.

## Revendications

1. Prothèse pour s'opposer au reflux gastrique dans l'oesophage, comportant un conduit tubulaire en un matériau polymère bio-compatible résistant à l'acide gastrique, dont une première extrémité est destinée à être fixée en amont de l'estomac et dont l'autre, libre, est destinée à occuper une position située en aval de ladite première extrémité, caractérisée par le fait que le conduit a une section de l'ordre de 25 à 30 mm d'une extrémité à l'autre et que sa longueur est comprise entre 5 et 10 cm, l'épaisseur de la paroi de ce conduit étant choisie pour permettre son écrasement sous une pression latérale exercée sur sa face externe et dont la valeur correspond à celle générée par un reflux gastrique.

2. Prothèse selon la revendication 1, caractérisée par le fait qu'elle présente à sa première extrémité une partie annulaire plus épaisse pour sa fixation.

3. Prothèse selon la revendication 1, caractérisée par le fait que l'épaisseur de la paroi dudit conduit est comprise entre 0,2 et 0,6 mm.

4. Prothèse selon la revendication 1, caractérisée par le fait qu'elle est en polyuréthane de qualité médicale.

5. Prothèse selon la revendication 1, caractérisée par le fait qu'elle est en silicone de qualité médicale.

6. Prothèse selon la revendication 1, caractérisée par le fait qu'elle est en PSE de qualité médicale.

7. Prothèse selon la revendication 1, caractérisée par le fait que ledit conduit tubulaire se divise longitudinalement en deux segments, un premier segment, adjacent à la première extrémité du conduit présentant une conicité de l'ordre de 3 ° et un second segment dant la conicité est de l'ordre de 1°, l'épaisseur de paroi du second segment étant inférieur à celle du premier.

8. Prothèse selon la revendication 7, caractérisée par le fait que ledit second segment représente environ 2/3 de la longueur totale du conduit.

9. Prothèse selon la revendication 7, caractérisée par le fait que l'épaisseur de paroi dudit segment est de l'ordre de la moitié de celle du premier segment.

## Claims

1. A prosthesis to prevent gastric reflux in the esophagus including a tube made of a biocompatible polymer that is resistant to gastric acids, one end of which is implanted at the upper opening of the stomach while the other hangs freely below it in the stomach cavity, characterized in that the tube has a section diameter between 25 and 30 mm from one end to the other and that its length ranges between 5 and 10 cm, the thickness of the wall has been chosen to allow it to collapse under lateral pressure applied to its external wall, when the level of pressure generated by gastric reflux is reached.

2. Prosthesis as defined by claim 1, characterized by a thicker annular flange at its upper end for implantation.

3. Prosthesis as defined by claim 1, characterized in that the thickness of the wall of the tube ranges between 0.2 and 0.6 mm.

4. Prosthesis as defined by claim 1, characterized in that it is made in medical quality grade polyurethane.

5. Prosthesis as defined by claim 1, characterized in that it is made in medical quality grade silicone.

6. Prosthesis as defined by claim 1, characterized in that it is made in medical quality grade polystyrene-ethylene (PSE).

7. Prosthesis as defined by claim 1, characterized in that the tubular prosthesis is divided longitudinally in 2 segments, a first segment at the upper end of the prosthesis presenting a conicity of approximately 3° and a lower second segment with a conicity of approximately 1°, the thickness of the lower second segment being inferior to the thickness of the first upper segment.

8. Prosthesis as defined by claim 7, characterized in that the second segment represents approximately 2/3 of the total length of the tube.

9. Prosthesis as defined by claim 7, characterized in that the thickness of the second segment is approximately half of the segment of the first segment.

## Patentansprüche

1. Prothese zur Verhinderung des gastrischen Reflux in die Speiseröhre, bestehend aus einem röhrenförmigen Element aus biokompatiblem Polymer-Werkstoff, der gegen Magensäure resistent ist. Das Röhrenelement wird an einem Ende oberhalb des Mageneingangs befestigt, während das andere, unbefestigte Ende dazu bestimmt ist, eine in Flußrichtung tiefer liegende Position einzunehmen. Das Röhrenelement hat durchgehend einen Durchschnitt in der Größenordnung von 25 bis 30 mm und ist 5 bis 10 cm lang. Seine Wandstärke wird so gewählt, daß das Element unter seitlicher Druckeinwirkung zusammengequetscht werden kann, die dem Druck des gastrischen Reflux entspricht.

2. Prothese nach Patentanspruch 1, gekennzeichnet durch die Tatsache, daß ein Ende eine verdickte Ringpartie aufweist, die zur Befestigung dient.

3. Prothese nach Patentanspruch 1, gekennzeichnet durch die Tatsache, daß die Wandstärke zwischen 0,2 und 0,6 mm beträgt.

4. Prothese nach Patentanspruch 1, gekennzeichnet durch die Tatsache, daß das Röhrenelement aus für Medizinzwecke geeignetem Polyurethan-Werkstoff hergestellt wurde.

5. Prothese nach Patentanspruch 1, gekennzeichnet durch die Tatsache, daß das Röhrenelement aus für Medizinzwecke geeignetem Silikon-Werkstoff hergestellt wurde.

6. Prothese nach Patentanspruch 1, gekennzeichnet durch die Tatsache, daß das Röhrenelement aus für Medizinzwecke geeignetem PSE-Werkstoff hergestellt wurde.

7. Prothese nach Patentanspruch 1, gekennzeichnet durch die Tatsache, daß das Röhrenelement sich in der Länge in zwei Segmente aufteilt, wobei das erste Segment neben dem ersten Ende des Elements liegt und eine Konizität von 3° aufweist, und das zweite Segment mit einer Konizität von 1° sowie mit einer kleineren Wandstärke des zweiten Segments im Vergleich zum ersten.

8. Prothese nach Patentanspruch 7, gekennzeichnet durch die Tatsache, daß das zweite oben beschriebenen Segment etwa 2/3 (zwei Drittel) der Gesamtlänge des Röhrenelements hat.

9. Prothese nach Patentanspruch 7, gekennzeichnet durch die Tatsache, daß die Wandstärke des oben beschriebenen zweiten Segments etwa halb so groß ist wie die des ersten Segments.
